# EUROPÄISCHE PATENTSCHRIFT

(11) **EP 1 454 650 B1**
(45) Veröffentlichungstag und Bekanntmachung des Hinweises auf die Patenterteilung: **10.10.2007**
(21) Anmeldenummer: 04003093.4
(22) Anmeldetag: 12.02.2004
(51) Int. Cl.: A61M 39/22, F16K 11/07, A61J 1/00

(54) **Transfervorrichtung, insbesondere für medizinische Fluids**
Transfer device, in particular for medical fluids
Dispositif de transfer, notamment pour des fluides médicaux

(30) Priorität: 06.03.2003 DE 10310110
(43) Veröffentlichungstag der Anmeldung: 08.09.2004
(73) Patentinhaber: CSL Behring GmbH, 35041 Marburg (DE)
(72) Erfinder: Jansen, Hubert, Dr., 52223 Stolberg (DE); Wortmann, Uwe, 35043 Marburg (DE)

(56) Entgegenhaltungen:
- EP-B- 0 521 460
- WO-A-97/20536
- DE-A- 10 057 153
- US-A- 4 423 741
- US-A- 5 971 021
- US-B1- 6 240 960
- US-B1- 6 355 023
- US-B1- 6 379 340

## Beschreibung

Die Erfindung betrifft eine Transfervorrichtung, insbesondere für medizinische Fluids mit einem Gehäuse, einem im Gehäuse gelagerten ersten nadel- oder dornförmigen Einstechelement, einem im Gehäuse gelagerten zweiten, nadel- oder dornförmigen Einstechelement, wobei die beiden Einstechelemente voneinander weg gerichtet sind und Strömungskanäle aufweisen, ferner mit einem zwischen den beiden Einstechelementen angeordneten Schieber, der im Gehäuse gelagert und zu den Einstechelementen verschiebbar ist, wobei in einer ersten Verschiebestellung des Schiebers eine Strömungsverbindung zwischen den beiden Einstechelementen und in einer zweiten Verschiebestellung des Schiebers eine Strömungsverbindung zwischen einem der Einstechelemente und einer seitlichen Öffnung des Gehäuses gebildet ist.

In der Medizintechnik ist es erforderlich, Flüssigkeit von einem ersten Behältnis, insbesondere einem Fläschchen, zu einem zweiten Behältnis, das eine zu lösende Substanz aufweist, insbesondere einem Fläschchen, überzuleiten. Hierzu wird eine Transfervorrichtung mit den Einstechelementen, bei denen es sich um nadel- oder dornförmige Elemente handelt, in die die beiden Behältnisse jeweils verschließenden und aus Gummi oder dergleichen bestehenden Verschlussstopfen eingestochen, d. h. die Verschlussstopfen mittels der Einstechelemente durchstochen, so dass in der ersten Verschiebestellung des Schiebers eine Fließverbindung zwischen den beiden Behältnissen über die Strömungskanäle geschaffen wird. Der Übergang der Flüssigkeit wird insbesondere dadurch gewährleistet, dass im Inneren des die zu lösende Substanz aufnehmenden Behältnisses Vakuum herrscht. Sind durch die Einstechelemente weitere Strömungskanäle geführt, können diese, sofern kein Vakuum in dem genannten Behältnis herrscht, dem Druckausgleich zwischen den beiden Behältnissen dienen. Das Überleiten der Flüssigkeit erfolgt mit nach oben gehaltenem, die Flüssigkeit aufnehmenden Behältnis, so dass die Flüssigkeit in das unten befindliche, die zu lösende Substanz aufnehmende Behältnis übertreten kann. Nach dem Lösen der Substanz wird der Schieber in die zweite Verschiebestellung überführt und damit die Verbindung zwischen den beiden Behältnissen gesperrt. Dann wird die Transfervorrichtung gedreht, so dass das Behältnis mit der aufgelösten Substanz oben ist. Da dasjenige Einstechelement, das in den Stopfen des oben angeordneten Behältnisses eingesteckt ist, sich nun in Fließverbindung mit einem seitlichen Entnahmekanal befindet, kann die gelöste Flüssigkeit mittels einer Spritze der Flasche entnommen werden.

Eine solche Transfervorrichtung ist nicht nur zum Lösen eines Medikamentes geeignet, sondern auch zum Mischen zweier Flüssigkeiten, zum Überleiten eines Gases usw.

Eine Transfervorrichtung der eingangs genannten Art ist aus der EP 0 521 460 A1 bekannt. Dort sind die beiden Einstechelemente sowohl mit einem Flüssigkeitskanal als auch mit einem Luftkanal versehen. Mit dem Flüssigkeitskanal wirkt ein Schieber zusammen, der unmittelbar der Aufnahme des Spritzenkonus einer Spritze dient. Der Schieber ist verschiebbar, derart, dass der Flüssigkeitskanal in der ersten Verschiebestellung des Schiebers frei durchgängig ist. In den Entnahmekanal des Schiebers ist ein Lippenventil eingesetzt. Dieses verhindert, dass Flüssigkeit nach außen austritt, insbesondere dann, wenn keine Spritze in den Schieber eingesetzt ist. In der zweiten Verschiebestellung des Schiebers verschließt ein vorspringender Ansatz des Schiebers den Zugang zum Strömungskanal desjenigen Einstechelementes, das in das Behältnis eingesetzt ist, welches ursprünglich die Flüssigkeit zum Lösen der Substanz aufnahm. Aufgrund dieser Position des Schiebers kann mittels der Spritze, am Lippenventil vorbei, die gelöste Substanz dem anderen Behältnis entnommen werden. Zur Führung des Schiebers im Gehäuse weist dieser einen zylindrischen Ansatz auf, der mit einem entsprechenden zylindrischen Ansatz des Gehäuses zusammenwirkt.

Nachteilig ist bei dieser Transfervorrichtung deren komplizierter Aufbau. So ist ein besonders gestaltetes Ventil vorzusehen, mit dem der Schieber zu bestücken ist. Entsprechend ist der Schieber konstruktiv aufwendig auszubilden, abgesehen von dem Erfordernis, dass er den Vorsprung besitzt, mit dem der Flüssigkeitskanal absperrbar ist. Beim Handling ist von besonderem Nachteil, dass die Spritze mit einem beweglichen Teil, dem Schieber, verbunden wird. Es besteht somit die Gefahr, dass beim Entnehmen der Flüssigkeit versehentlich der Schieber in die erste Verschiebestellung überführt wird.

Aus der US 6,379,340 B1 ist eine Transfervorrichtung für medizinische Fluids bekannt, bei der statt eines Schiebers ein in einem Gehäuse drehbar gelagertes Teil Verwendung findet. Das Teil bildet in einer ersten Drehstellung eine Strömungsverbindung zwischen zwei Einstechelementen und in einer zweiten Drehstellung eine Strömungsverbindung zwischen einem der Einsteckelemente und einem seitlichen Entnahmekanal des drehbaren Elementes. Das drehbare Element weist einen Anschluss auf, in den der Anschlussstutzen einer Spritze einsteckbar ist. Bei dieser Transfervorrichtung ist der bauliche Aufwand sowie der Montageaufwand erheblich. Überdies ist das Handling nicht zufriedenstellend, weil aufgrund des Umstandes, dass zum Überführen des Transfersets in die Betriebsstellungen das drehbare Teil gedreht werden muss, dem Anwender in aller Regel nicht klar ist, in welcher funktionellen Stellung sich die Transfervorrichtung befindet und in welche Richtung das Teil zu drehen ist.

In der US 6,378,714 B1 ist eine Transfervorrichtung mit einem Einstechelement beschrieben. Ein Fläschchen ist mittels eines Gummistopfens verschlossen, ein Gehäuse ist auf das verschlossene Fläschchen aufgesteckt. Es nimmt einen Schieber mit Einstechelement auf. Beim Einstecken des Konus einer Spritze in das Gehäuse wird der in dem Gehäuse geführte Schieber verschoben, und es durchsticht das Einstechelement den Verschluss des Fläschchens. Durch einen im Einstechelement und dem Schieber gebildeten Kanal kann das Fluid strömen. Diese Transfervorrichtung weist keine unterschiedlichen Strömungsverbindungen, einerseits zwischen zwei Einsteckelementen, andererseits einem Einsteckelement und einer seitlichen Eintrittsöffnung des Gehäuses, auf.

D4 (US 4,423,741) beschreibt eine Transfervorrichtung zum Sammeln von Mittelstrahlurin, wobei die Vorrichtung zwei Strömungskanäle hat, die durch einen Teil verbunden sind, in dem eine Strömungsverbindung zwischen den Strömungskanälen und einer seitlichen Entnahmeöffnung durch einen Schieber gezielt hergestellt werden kann.

Aufgabe der Erfindung ist es, eine Transfervorrichtung der eingangs genannten Art so weiterzubilden, die vom Benutzer eindeutig und sicher bedient werden kann. Sie soll zudem einfach und kostengünstig herstell- und montierbar sein.

Gelöst wird die Aufgabe durch eine Transfervorrichtung gemäß Anspruch 1.

Erfindungsgemäß ist somit vorgesehen, dass der Schieber zu dem Zeitpunkt, zu dem die Spritze mit dem Gehäuse verbunden wird, aufgrund der Bewegung der Spritze im Gehäuse verschoben wird und hierdurch den anderen Funktionszustand der Transfervorrichtung herbeiführt. In diesem Zustand nimmt der Schieber die zweite Verschiebestellung ein, in der er die Strömungsverbindung zwischen einem der Einstechelemente und der seitlichen Öffnung des Gehäuses schafft.

Bei der erfindungsgemäßen Transfervorrichtung ist das Handling besonders einfach, weil der Vorgang des Montierens der Spritze am Gehäuse zwangsläufig zum Überführen des Schiebers in die gewünschte Stellung führt. Der Spritzenkonus kontaktiert beim Einstecken der Spritze stirnseitig den Schieber und verschiebt diesen. Ein Verschieben des Schiebers in dessen erste Verschiebestellung ist bei in das Gehäuse eingesteckter Spritze unmöglich. Dadurch, dass die Spritze in das Gehäuse eingesteckt ist, ist jedes nachteilige, unsachgemäße Handling ausgeschlossen, das sich insbesondere beim Stand der Technik ergibt, bei dem der Spritzenkonus in den Schieber bzw. in das drehbar gelagerte Teil eingesteckt ist.

Der bauliche Aufwand und der Montageaufwand sind bei der erfindungsgemäßen Transfervorrichtung minimal, da der Schieber einteilig gestaltet werden kann und nur axial im Gehäuse zu führen ist.

Die Transfervorrichtung kann auf unterschiedliche Art und Weise gestaltet sein. Als bevorzugt wird es angesehen, wenn sie nur zwei Einstechelemente mit jeweils einem Strömungskanal aufweist. Die Transfervorrichtung findet somit im Zusammenhang mit der Überleitung eines Fluids in einen unter Vakuum stehenden Behälter Verwendung. Die Einstechelemente sind als Nadeln oder Dorne ausgebildet. Der Widerstand zum Durchdringen der die Behältnisse verschließenden Stopfen ist somit sehr gering.

Vorzugsweise lässt sich die Spritze im Bereich des Konus fest mit dem Anschluss des Gehäuses verbinden. Unter diesem Aspekt ist der Anschluss insbesondere als weiblicher Luer-Anschluss bzw. Luer-Lock-Anschluss ausgebildet.

In der ersten Verschiebestellung des Schiebers ist noch keine Spritze mit dem Gehäuse verbunden. Zu diesem Zeitpunkt verschließt vorzugsweise ein Verschlusselement den Anschluss. Hierbei handelt es sich insbesondere um einen Deckel, der mit dem Anschluss des Gehäuses verschraubbar ist. Um Leckagen auszuschließen, sollte das Verschlusselement den Anschluss dichtend verschließen. Ist der Anschluss mit einem Gewinde versehen, um beispielsweise die Spritze dort mit dem Anschluss verbinden zu können, ist gleichfalls die Verschlusskappe mit einem Gewinde versehen, so dass sie in der Funktionsstellung des Transfersets mit in erster Verschiebestellung befindlichem Schieber auf den Anschluss des Gehäuses aufgeschraubt werden kann.

Als besonders vorteilhaft wird es angesehen, wenn der Schieber im Gehäuse geführt ist. Hierbei sind insbesondere eine präzise Abdichtung des Schiebers zum Gehäuse vorgesehen, insbesondere in denjenigen Bereichen, die aus dem Gehäuse heraus führen. Der Schieber lässt sich optimal abdichten, wenn er stiftförmig ausgebildet ist. Diese Gestaltung ermöglicht eine besonders einfache Montage des Stiftes im Gehäuse.

Eine bevorzugte Gestaltung des Schiebers sieht vor, dass er im Bereich seines Umfanges einen sich zumindest über einen Teilkreis erstreckenden Verbindungskanal aufweist, der in der ersten Verschiebestellung des Schiebers die Strömungskanäle der beiden Einsteckelemente miteinander verbindet. Aus Herstellungs- und Montagegründen ist anzustreben, den Schieber so weit wie möglich als rotationssymmetrisches Teil auszubilden. Unter diesem Aspekt wird es als vorteilhaft angesehen, wenn sich der Verbindungskanal über den gesamten Umfang des Schiebers erstreckt.

In der zweiten Verschiebestellung des Schiebers kann das Fluid auf besonders einfache Art und Weise abgeführt werden, wenn der Schieber einen sich in Längsrichtung des Schiebers erstreckenden Entnahmekanal aufweist, der sich in der zweiten Verschiebestellung des Schiebers in Strömungsverbindung mit dem Strömungskanal eines der Einstechelemente befindet. Insofern ist der Schieber, in Abstand zum Verbindungskanal, mit dem senkrecht hierzu verlaufenden Entnahmekanal zu versehen. Der Entnahmekanal ist zweckmäßig durch einen Außenschlitz des Schiebers gebildet und besitzt insbesondere V-förmigen Querschnitt. Das Fluid strömt in diesem Fall somit nicht durch den Schieber, sondern zwischen ihm und dem Gehäuse.

Gemäß einer ersten grundsätzlichen Ausführungsform der Transfervorrichtung ist vorgesehen, dass der Schieber starr ist. Insbesondere besteht das Gehäuse und der Schieber aus Kunststoff. Die Strömungskanäle in den Einstechelementen setzen sich durch das Gehäuse zum Schieber fort. Beim Einsetzen der Spritze in das Gehäuse entspricht der Verschiebeweg des Schiebers bei Kontakt der Spritze am Schieber der Einsteckdistanz der Spritze in das Gehäuse. Bei dieser Ausführungsform lässt sich der Schieber besonders einfach montieren, wenn das Gehäuse auf der dem Anschluss für die Spritze abgewandten Seite eine Öffnung zum Einführen des Schiebers aufweist. Zwischen dem Schieber und dem Gehäuse wirksame Rastmittel sollten zumindest in der ersten Verschiebestellung diese Teile zueinander positionieren. Es ist damit sichergestellt, dass der Schieber nur bei Einwirkung der Spritze in die zweite Verschiebestellung überführt werden kann. Sofern keine von außerhalb des Gehäuses bedienbaren Verstellmittel auf den Schieber einwirken, kann dieser nicht von der zweiten Verschiebestellung in die erste Verschiebestellung zurückgeschoben werden. - Dies ist bei einer grundsätzlichen zweiten Ausführungsform des Transfersets möglich.

Gemäß dieser zweiten Ausführungsform ist vorgesehen, dass der Schieber zumindest in einem Teilbereich elastisch ausgebildet ist, insbesondere aus Gummi besteht, wobei dieser Teilbereich beim Verschieben des Schiebers von der ersten Verschiebestellung gegen einen gehäuseseitigen Anschlag in eine zweite Verschiebestellung verformt wird. Vorzugsweise ist der Schieber insgesamt elastisch ausgebildet.

Bei dieser Gestaltung des Schiebers und des Gehäuses ergibt sich ein Federeffekt, weil der Schieber beim Überführen in die zweite Verschiebestellung infolge Anlage am gehäuseseitigen Anschlag komprimiert wird. Wird somit die Spritze wieder aus dem Gehäuse herausgezogen, folgt der Schieber, da er sich in seine Längsrichtung entspannt, der Bewegung der Spritze und nimmt, bei abgenommener Spritze, die erste Verschiebestellung wieder ein.

Die Ausbildung des Schiebers als zumindest in einem Teilbereich elastisches Bauteil beinhaltet den besonderen Vorteil, dass der verformte Bereich des Schiebers Dichtkräfte auf die Innenwandungen des Gehäuses überträgt, womit Leckagen der Transfervorrichtung wirksam vermieden werden.

Bei dieser Ausführungsform lässt sich der Schieber besonders einfach montieren, wenn das Gehäuse auf der dem Anschluss zugewandten Seite eine Öffnung zum Einführen des Schiebers aufweist. Die der Öffnung abgewandte Seite des Gehäuses bildet den Anschlag für den Schieber. Es wird als besonders vorteilhaft angesehen, wenn zumindest derjenige Bereich des Schiebers elastisch ausgebildet ist, der dem Verbindungskanal zugeordnet ist. Dort weist der Schieber somit eine verminderte Dicke auf, im Sinne des vorbeschriebenen, sich über den gesamten Umfang des Schiebers erstreckenden Verbindungskanals.

Um ein einfaches Handling der mit der Transfervorrichtung zusammenwirkenden Behältnisse, insbesondere der Fläschchen, zu gewährleisten, sollte das Gehäuse im Bereich der Einstechelemente zylindrische Aufnahmen zum rastierenden Einstecken der Behältnisse bzw. des Halses des jeweiligen Fläschchens aufweisen.

Um eine größtmögliche Partikelfreiheit des in die Spritze aufgezogenen Fluids zu gewährleisten, wird es als vorteilhaft angesehen, wenn in den Strömungsweg von dem mit dem Entnahmekanal verbindbaren Einstechelement zum Entnahmekanal ein Filterelement integriert ist.

Weitere Merkmale der Erfindung sind in den Patentansprüchen, der Beschreibung der Figuren und den Figuren selbst darstellt.

In der Zeichnung der Figuren ist die Erfindung anhand zweier Ausführungsbeispiele veranschaulicht, ohne hierauf beschränkt zu sein. Es zeigt:
- Fig. 1: eine räumliche Ansicht der Transfervorrichtung mit zwei in diese eingesteckten Fläschchen,
- Fig. 2.: eine räumliche Ansicht der Transfervorrichtung,
- Fig. 3: für eine erste Ausführungsform der Transfervorrichtung einen Längsschnitt durch diese, der durch den Mittelpunkt des Radius der Einstechelemente und die Längsmittelachse des Schiebers geführt ist,
- Fig. 4: einen Schnitt durch die Transfervorrichtung gemäß Figur 3 mit zusätzlich veranschaulichten, in diese eingesetzten verschlossenen Fläschchen, in der ersten Verschiebestellung des Schiebers,
- Fig. 5: einen Schnitt gemäß Figur 4, bei um die Längsmittelachse des Schiebers um 180° geschwenkter Transfervorrichtung, veranschaulicht für die zweite Verschiebestellung des Schiebers und in das Gehäuse eingesteckter Spritze,
- Fig. 6: eine räumliche Ansicht des bei der Ausführungsform nach den Figuren 3 bis 5 Verwendung findenden Schiebers,
- Fig. 7: eine zweite Ausführungsform der Transfervorrichtung mit in diese eingesetzten Fläschchen, in einer Schnittdarstellung gemäß Figur 5, jedoch mit in erster Verschiebestellung befindlichem Schieber,
- Fig. 8: eine Schnittdarstellung der zweiten Ausführungsform gemäß Figur 7, veranschaulicht bei in das Gehäuse eingeführter Spritze und in zweiter Verschiebestellung befindlichem Schieber,
- Fig. 9: eine räumliche Ansicht des bei der zweiten Ausführungsform gemäß der Figuren 7 und 8 Verwendung findenden Schiebers.

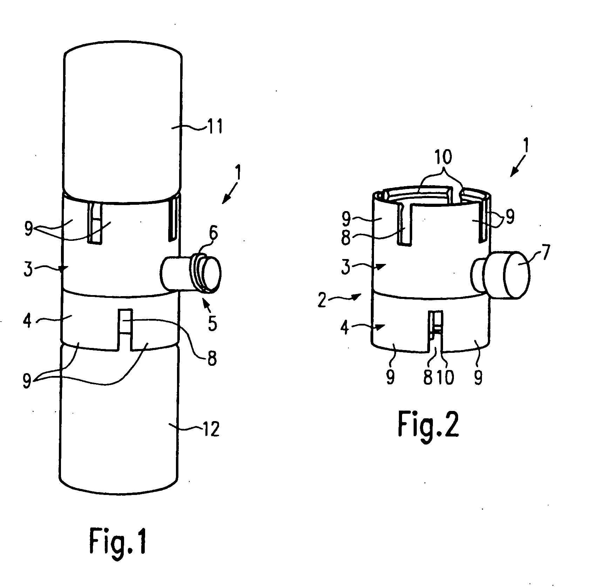

Bei der in den Figuren 1 und 2 veranschaulichten Transfervorrichtung 1 ist das Gehäuse 2 durch zwei ineinander gesteckte und miteinander verbundene Gehäusehälften 3 und 4 gebildet. Die Gehäusehälfte 3 weist einen seitlichen Anschluss 5 mit Außengewinde 6 zum Aufschrauben einer Verschlusskappe 7 bzw. einer Entnahmespritze auf. Im Bereich der freien Enden weisen die Gehäusehälften 3 und 4 sich in Längsrichtung des Gehäuses 2 erstreckende Schlitze 8 auf, die zwischen sich Gehäuselappen 9 bilden, die im Bereich ihrer freien Enden mit nach innen gerichteten Rastvorsprüngen 10 versehen sind. In die jeweilige Gehäusehälfte 3 bzw. 4 ist ein Glasfläschchen 11 bzw. 12 eingesetzt und mittels der Rastvorsprünge gehalten. Im Ausgangszustand nimmt das Fläschchen 11 beispielsweise eine medizinische Flüssigkeit, das Fläschchen 12 eine zu lösende Substanz auf. Vor dem Einsetzen des Fläschchens 12 in die Transfervorrichtung 1 herrscht in dessen Innenraum insbesondere Vakuum.

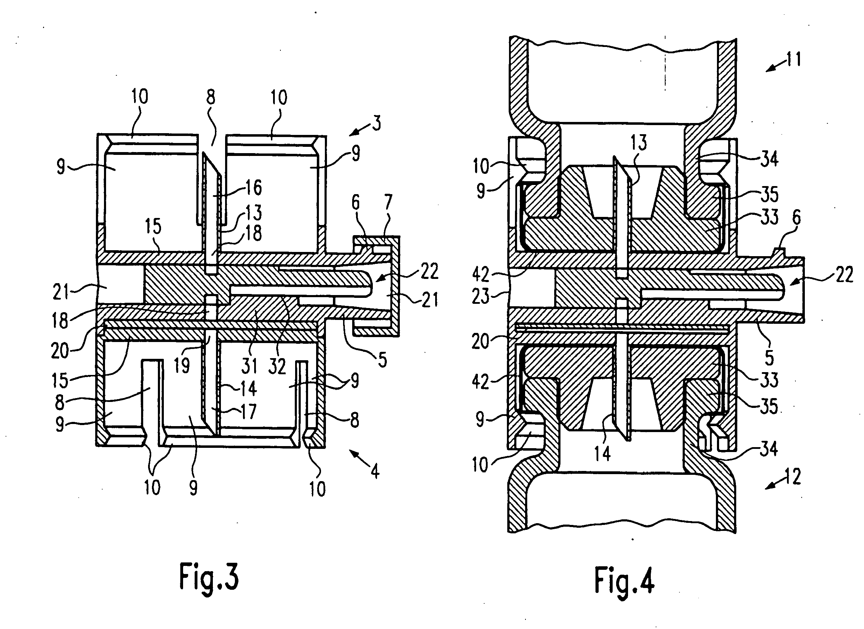

Der Aufbau einer ersten Ausführungsform der Transfervorrichtung ist in Figur 3 veranschaulicht. Das jeweilige Gehäuseteil 3 bzw. 4 besteht aus Kunststoff und bildet ein Bauteil mit einem Einstechdorn 13 bzw. 14, der im Bereich des Mittelpunktes der jeweiligen Grundplatte 15 des Gehäuseteils 3 bzw. 4 mit dieser Grundplatte 15 verbunden ist. Die Länge des jeweiligen Einstechdornes 13 bzw. 14 ist so bemessen, dass er bis zu den Rastvorsprüngen 10 reicht. Der im jeweiligen Einstechdorn 13 bzw. 14 gebildete Strömungskanal 16 bzw. 17 setzt sich gemäß den Abschnitten 18 bzw. 19 im Gehäuseteil 3 bzw. 4 fort. Dort wo die beiden Gehäuseteile 3 und 4 ineinander gesteckt und miteinander fest verbunden sind, ist ein flacher Raum gebildet, in den ein Filterelement 20 eingesetzt ist.

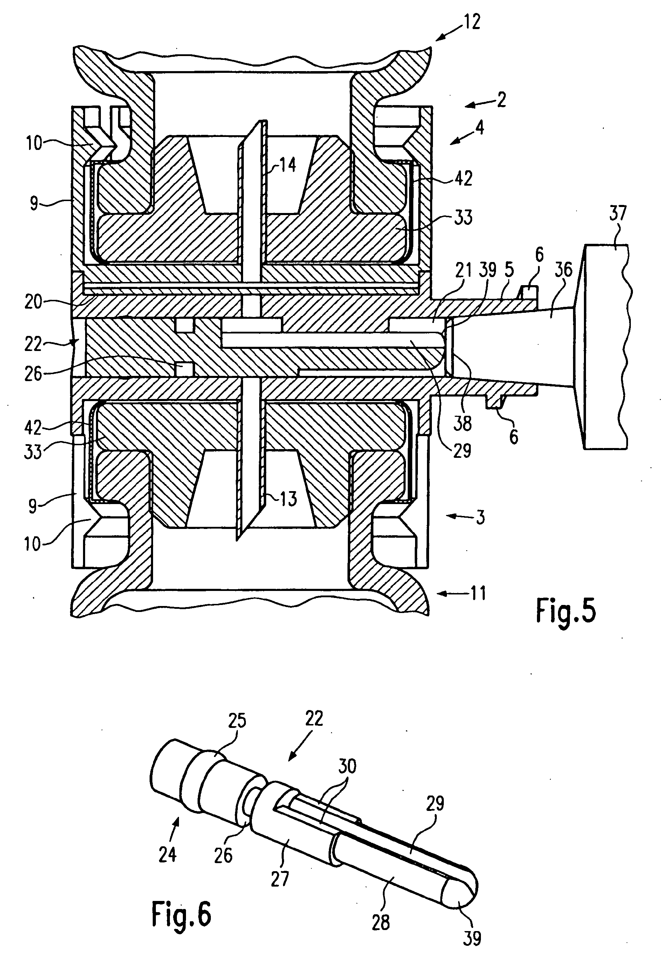

Bezogen auf die Längsorientierung des Gehäuses 2, somit die Längsorientierung der Einstechdorne 13 und 14, ist senkrecht zu diesen das Gehäuseteil 3 mit einem Durchgang 21 versehen. In diesen ist, von der dem Anschluss 5 abgewandten Seite, ein Schieber 22 eingesteckt, der in Figur 6 im Detail veranschaulicht ist. Dieser ist im Wesentlichen rotationssymmetrisch ausgebildet. So weist der, der hinteren Öffnung des Gehäuseteiles 3 zugewandte rotationssymmetrische Abschnitt 24 des Schiebers 22 einen Wulst 25 auf, der in diesem Bereich gegen die Wandung des Durchgangs 21 drückt und bewirkt, dass der Schieber 22 nur bei Einleiten einer ausreichend hohen Axialkraft in diesen verschoben werden kann. Andererseits dichtet dieser Wulst 25 den Schieber 22 gegenüber dem Durchgang 21 des Gehäuseteils 3 ab. An das vordere Ende des rotationssymmetrischen Abschnittes 24 schließt sich im Schieber 22 eine umlaufende Nut 26 an. Von dort, nach vorne, schließt sich ein im Wesentlichen rotationssymmetrischer Abschnitt 27 an, dessen Durchmesser demjenigen des rotationssymmetrischen Abschnittes 24, somit desjenigen neben dem Wulst 25 entspricht. Schließlich schließt sich vorne an den Abschnitt 27 ein im Wesentlichen rotationssymmetrischer Abschnitt 28 an, der einen geringeren Durchmesser aufweist als der rotationssymmetrische Abschnitt 27. Die Abschnitte 27 und 28 sind mit einem V-förmigen Schlitz 29 versehen, der in Abstand von der Nut 26 endet. Der Abschnitt 27 ist im Bereich des V-förmigen Schlitzes 29 abgeflacht, so dass sich dort beidseits des Schlitzes 29 zwei Führungsflächen 30 ergeben. Entsprechend, wie es der Darstellung der Figur 3 zu entnehmen ist, wirkt ein in den Durchgang 21 ragender Vorsprung 31 mit seiner Führungsfläche 32 mit der Führungsfläche 30 des Schiebers 22 zusammen. Infolgedessen kann der Schieber nur in seiner Längsrichtung verschoben, nicht aber geschwenkt werden.

Die Funktion der insoweit beschriebenen Transfervorrichtung stellt sich wie folgt dar:

Figur 3 zeigt die Transfervorrichtung 1 in der ersten Verschiebestellung des Schiebers 22. Hierbei fluchtet die Nut 26 des Schiebers 22 mit den Abschnitten 18 im Gehäuseteil 3; die Nut 26 steht somit in Strömungsverbindung mit den Strömungskanälen 16 und 17 der beiden Einstechdorne 13 und 14. Der Anschluss 5 ist mittels der Kappe 7 dicht verschlossen. Hiervon ausgehend wird zunächst das die Flüssigkeit enthaltende Fläschchen in das Gehäuseteil 3 eingesteckt. In die Öffnung des Fläschchens 11 ist ein elastischer Stopfen 33 eingesteckt, der vom Einstechdorn 13 durchstochen wird. In der in das Gehäuseteil 3 eingesetzten Stellung des Fläschchens 11 hintergreifen die Rastvorsprünge 10 dieses Gehäuseteils 3 den sich an den Flaschenhals 34 anschließenden stopfenseitigen Wulst 35 des Fläschchens 11. Vor dem Verbinden des unter Vakuum stehenden und das zu lösende Medikament aufnehmenden Fläschchens 12 mit der Transfervorrichtung 1, wird diese in der in den Figuren 3 und 4 gezeigten Position platziert, somit mit oben angeordnetem Gehäuseteil 3 und oben befindlichem Fläschchen 11, das die Flüssigkeit aufnimmt. Dann wird das andere Fläschchen 12 in das Gehäuseteil 4 entsprechend eingeführt, wobei der Einstechdorn 14 den Stopfen 33, der dieses Fläschchen 12 verschließt, durchdringt. Bei vollständig in das Gehäuseteil 4 eingeführtem Fläschchen 12 hintergreifen die diesem Gehäuseteil zugeordneten Rastvorsprünge 10 den sich an den Flaschenhals 34 dieses Fläschchens anschließenden Wulst 35. Da die Einstechdorne 13 und 14 in die Innenräume der Fläschchen 11 und 12 ragen, wird, aufgrund des Unterdrucks im Fläschchen 12, die Flüssigkeit vom Fläschchen 11 über den Strömungskanal 16, die Nut 26 und den Strömungskanal 17 in das Fläschchen 12 überführt und die dort befindliche Substanz gelöst.

Zum seitlichen Entnehmen der gelösten Substanz aus der Transfervorrichtung 1 wird zunächst, wie es zur Figur 4 veranschaulicht ist, die den Schieberbereich auf der dem Wulst 25 abgewandten Seite abdichtende Verschlusskappe 7 vom Anschluss 5 abgeschraubt, womit das Vakuum abgebaut wird. Dann wird die Transfervorrichtung 1 mit den beiden in diese eingesetzten Fläschchen 11 und 12 um 180° um die Längsachse des Schiebers 22 in die in Figur 5 veranschaulichte Position geschwenkt. Infolgedessen befindet sich das Fläschchen 12 mit der darin befindlichen, gelösten Substanz oben und das leere Fläschchen 11 unten. Zum Entnehmen der gelösten Substanz wird, wie es in Figur 5 veranschaulicht ist, ein Spritzenkonus 36 einer nur teilweise dargestellten Spritze 37 in die konische Öffnung des Anschlusses 5 abgedichtet eingesteckt. Beim Einführen des Spritzenkonus 36 in den Anschluss 5 verschiebt das stirnseitige Ende 38 des Spritzenkonus 36 den Schieber 22, wobei der Spritzenkonus 36 ein sich verjüngendes Ende 39 des Schiebers 22 kontaktiert. In der vollständig in den Anschluss 5 eingesetzten Stellung des Spritzenkonus 36 nimmt der Schieber 22 die in Figur 5 gezeigte zweite Verschiebestellung ein, in der sich das der Spritze 37 abgewandte Ende des Schiebers 22 gerade noch vollständig innerhalb des Gehäuseteils 3 befindet, und nunmehr der Strömungskanal 17 des Einstechdorns 14 über die Abschnitte 18 und 19 in Strömungsverbindung mit dem V-förmigen Schlitz 29 im Schieber 22 steht, konkret in dem Bereich des V-förmigen Schlitzes, der in den Abschnitt 27 eingebracht ist. Beim Aufziehen der Spritze 37 kann somit über die Strömungsverbindung von Einstechdorn 14 und V-förmigem Schlitz 29 des Schiebers 22 das Fläschchen 12 entleert werden.

Bei der Ausführungsform nach den Figuren 3 bis 6 ist der Schieber starr ausgebildet, er besteht insbesondere aus Kunststoff.

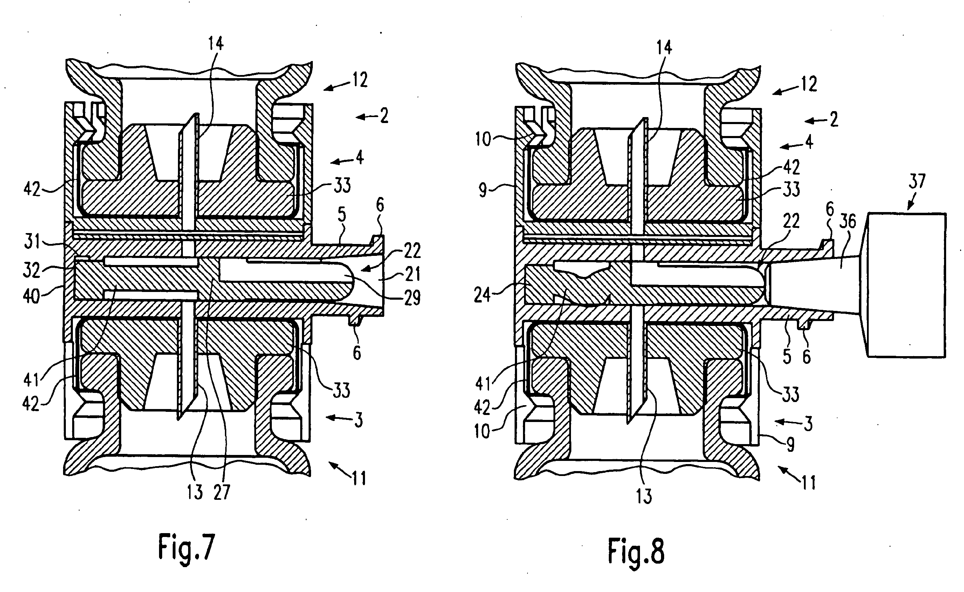

Eine weitere Ausführungsform der Transfervorrichtung 1 ist in den Figuren 7 bis 9 beschrieben. Diese unterscheidet sich von der ersten Ausführungsform nur hinsichtlich des Schiebers 22 und der Gestaltung des Gehäuseteils 3. Wegen der Übereinstimmung wird deshalb auf die vorgenannte Beschreibung verwiesen.

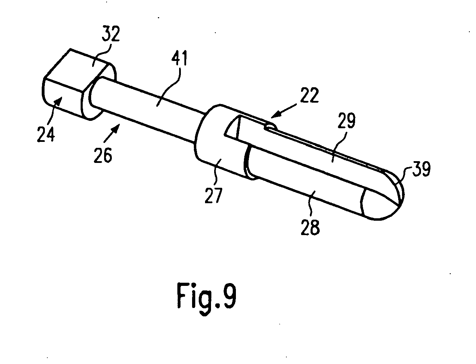

Bei der Ausführungsform nach den Figuren 7 bis 9 besteht der Schieber 22 insgesamt aus elastischem Material, insbesondere einem thermoplastischen Elastomer (TPE) bzw. Gummi. Der Schieber 22 wird über den Anschluss 5 in das Gehäuseteil 3 eingesetzt und es ist dieses im Bereich seines dem Anschluss 5 abgewandten Endes geschlossen. Diese Ausführungsform weist somit nicht die Öffnung 23 der ersten Ausführungsform auf. Bei der Ausführungsform nach den Figuren 7 bis 9 ist beim Schieber 22 der Abschnitt 27, der Abschnitt 28 und der V-förmige Schlitz 29 entsprechend ausgebildet. Die umlaufende Nut 26 ist wesentlich breiter ausgebildet als die Nut 26 der ersten Ausführungsform, in etwa so lang wie der Abschnitt 28. Entsprechend ist der Abschnitt 24 ohne Wulst 25 gestaltet und wesentlich kürzer als der Abschnitt 24 der ersten Ausführungsform. Der Abschnitt 24 weist zudem oben eine Abflachung 32 auf, die mit einer in dessen Bereich angeordneten Führungsfläche 31 des Gehäuseteils 3 zusammenwirkt. Der detaillierte Aufbau des Schiebers 22 dieser Ausführungsform ist in Figur 9 veranschaulicht.

Figur 7 zeigt diese Ausführungsform in der ersten Verschiebestellung des Schiebers, und zwar bereits in der nach oben geschwenkten Position des Fläschchens 12 mit der darin gelösten Substanz. Der Schieber 22 liegt an der dem Anschluss 5 abgewandten Gehäusewand 40 an. Die Strömungsverbindung der Strömungskanäle 16 und 17 der Einstechdorne 13 bzw. 14 erfolgt über die Nut 26. Zum Entnehmen der gelösten Substanz wird, wie es in Figur 8 veranschaulicht und zuvor ausführlich beschrieben ist, der Spritzenkonus 36 in den Anschluss 5 eingesteckt, wobei er den Schieber 22 stirnseitig kontaktiert und im Bereich des geschwächten Querschnittes 41, im Bereich dessen die Nut 26 definiert ist, aufgrund der elastischen Eigenschaft des Schiebers 22 verformt. Die Verformung in diesem Bereich des Schiebers 22 und infolgedessen die Verschiebung des dem Spritzenkonus 36 zugewandten Bereiches des Schiebers 22 in Richtung der Gehäusewand 40 bedingt, dass, wie es in Figur 8 veranschaulicht ist, der Strömungskanal 17 des Einstechdornes 14 in Strömungsverbindung mit dem V-förmigen Schlitz 29 gelangt.

Bedeutung kommt in diesem Zusammenhang dem vorzugsweise aus TPE geformten Schieber 22 zu. Dessen elastische Eigenschaft vereinfacht die Abdichtung des Schiebers 22 zum Gehäuseteil 3, so dass in den beiden Verschiebestellungen des Schiebers 22 eindeutige Flüssigkeitsströme, somit ohne Leckagen, durch die Transfervorrichtung geführt werden können. Der aus dem thermoplastischen Elastomer bestehende Schieber 22 hat einen Federeffekt, so dass sich das System automatisch verschließt, wenn die Spritze 37 aus dem Anschluss 5 herausgezogen wird. In diesem Fall wird der Schieber 22 umgehend in seine erste Verschiebestellung überführt.

Bei der ersten Ausführungsform nach den Figuren 3 bis 6, bei der der Schieber 22 starr ausgebildet ist, wird durch den Wulst 25 nicht nur die jeweilige Ausgangsstellung des Schiebers definiert, es wird vor allem der Durchgang 21 zu dem dem Anschluss 5 abgewandten Ende abgedichtet. Dies ist für die Funktion der Vorrichtung wesentlich. Würde diese Dichtung nicht funktionieren, würde das Vakuum in dem Fläschchen 12, das die zu lösende Substanz aufnimmt, abgebaut und ein Transfer möglich. Bei der Ausführungsform der nach den Figuren 7 bis 9 mit dem elastischen Schieber 22 und dem anders gestalteten Gehäuseteil 3 dichtet der elastische Schieber 22 aufgrund seiner Materialeigenschaften unmittelbar ab. Außerdem kann der elastische Schieber 22 von der Entnahmeseite eingebaut werden. Hierdurch kann auf den Durchgang 21 im Bereich des dem Anschluss 5 abgewandten Endes des Gehäuses 2 verzichtet werden, wodurch die Abdichtungsproblematik verringert wird.

Bei beiden Ausführungsformen wird durch das Verschließen des Strömungskanals 16 verhindert, dass Luft aus dem Fläschchen 11 angesaugt werden kann. Dies ist wichtig für die Entnahme der gelösten Substanz bzw. des gelösten Medikaments.

Mit der Spritze 37 wird bei der Entnahme ein Unterdruck erzeugt, wodurch die gelöste Substanz in die Spritze fliest. Wäre das Fläschchen 11 offen, so würde der in der Spritze 37 erzeugte Unterdruck verringert und so das Füllen der Spritze 37 erheblich erschwert.

Mit der Bezugsziffer 42 ist ein kappenartiger Folienverschluss bezeichnet, der den Wulst 35 des jeweiligen Fläschchens 11 bzw. 12 und den in diesen eingesteckten Stopfen 33 umgibt, womit der Stopfen sicher im Fläschchen gehalten ist.

### Bezugszeichenliste

- 1: Transfervorrichtung
- 2: Gehäuse
- 3: Gehäuseteil
- 4: Gehäuseteil
- 5: Anschluss
- 6: Gewinde
- 7: Verschlusskappe
- 8: Schlitz
- 9: Gehäuselappen
- 10: Rastvorsprung
- 11: Glasfläschchen
- 12: Glasfläschchen
- 13: Einstechdorn
- 14: Einstechdorn
- 15: Grundplatte
- 16: Strömungskanal
- 17: Strömungskanal
- 18: Abschnitt
- 19: Abschnitt
- 20: Filterelement
- 21: Durchführung
- 22: Schieber
- 23: Öffnung
- 24: Abschnitt
- 25: Wulst
- 26: Nut
- 27: Abschnitt
- 28: Abschnitt
- 29: Schlitz
- 30: Führungsfläche
- 31: Vorsprung
- 32: Führungsfläche
- 33: Stopfen
- 34: Flaschenhals
- 35: Wulst
- 36: Spritzenkonus
- 37: Spritze
- 38: Ende
- 39: Ende
- 40: Gehäusewand
- 41: Querschnitt
- 42: Folienverschluss

## Patentansprüche

1. Transfervorrichtung (1), insbesondere für medizinische Fluids mit einem Gehäuse (2), einem im Gehäuse (2) gelagerten ersten, nadel- oder dornförmigen Einstechelement (13), einem im Gehäuse (2) gelagerten zweiten, nadel- oder dornförmigen Einstechelement (14), wobei die beiden Einstechelemente (13, 14) voneinander weg gerichtet sind und Strömungskanäle (16, 17) aufweisen, ferner mit einem zwischen den beiden Einstechelementen (13, 14) angeordneten Schieber (22), der im Gehäuse (2) gelagert und zu den Einstechelementen (13, 14) verschiebbar ist, wobei in einer ersten Verschiebestellung des Schiebers (22) eine Strömungsverbindung zwischen den beiden Einstechelementen (13, 14) und in einer zweiten Verschiebestellung des Schiebers (22) eine Strömungsverbindung zwischen einem der Einstechelemente (14) und einer seitlichen Öffnung (21) des Gehäuses (2) gebildet ist, wobei das Gehäuse (2) im Bereich der seitlichen Öffnung (21) einen Anschluss (5) zum Einführen eines Spritzenkonus (36) einer Spritze (37) aufweist, wobei der Schieber (22) beim Einstecken des Spritzenkonus (36) in den Anschluss (5) durch das stirnseitige Ende (38) des Spritzenkonus (36) von der ersten Verschiebestellung in die zweite Verschiebestellung verschoben wird, **dadurch gekennzeichnet, dass** der Schieber (22) im Bereich seines Umfanges einen sich zumindest über einen Teilkreis erstreckenden Verbindungskanal (26) aufweist, der in der ersten Verschiebestellung des Schiebers (22) die Strömungskanäle (16, 17) der beiden Einsteckelemente (13, 14) miteinander verbindet und dass der Schieber (22) am Umfang einen sich in Längsrichtung des Schiebers (22) erstreckenden Entnahmekanal (29) aufweist, der sich in der zweiten Verschiebestellung des Schiebers (22) in Strömungsverbindung mit dem Strömungskanal (17) eines der Einsteckelemente (13, 14) befindet, wobei der Entnahmekanal (29) durch einen Außenschlitz des Schiebers (22) gebildet ist und insbesondere V-förmigen Querschnitt aufweist.

2. Vorrichtung nach Anspruch 1, **dadurch gekennzeichnet, dass** der Anschluss (5) als weiblicher Luer-Anschluss ausgebildet ist.

3. Vorrichtung nach Anspruch 1 oder 2, **dadurch gekennzeichnet, dass** der Anschluss (5) mittels eines Verschlusselementes (7) verschließbar ist, insbesondere dichtend verschließbar ist.

4. Vorrichtung nach Anspruch 3, **dadurch gekennzeichnet, dass** das Verschlusselement (7) als Verschlusskappe ausgebildet ist, die auf den Anschluss (5) aufschraubbar ist.

5. Vorrichtung nach einem der Ansprüche 1 bis 4 **dadurch gekennzeichnet, dass** der Schieber (22) verdrehsicher im Gehäuse (2) gelagert ist.

6. Vorrichtung nach einem der Ansprüche 1 bis 5, **dadurch gekennzeichnet, dass** der Schieber (22) geklemmt oder zumindest in seiner ersten Verschiebestellung rastierend im Gehäuse (2) positioniert ist.

7. Vorrichtung nach einem der Ansprüche 1 bis 6, **dadurch gekennzeichnet, dass** das Gehäuse (2) auf der dem Anschluss (5) abgewandten Seite eine Öffnung (23) zum Einführen des Schiebers (22) aufweist.

8. Vorrichtung nach einem der Ansprüche 1 bis 7, **dadurch gekennzeichnet, dass** der Schieber (22) zumindest in einem Teilbereich (41) elastisch ausgebildet ist, insbesondere aus einem thermoplastischen Elastomer oder aus Gummi besteht, wobei dieser Teilbereich (41) beim Verschieben des Schiebers (22) von der ersten Verschiebestellung gegen einen gehäuseseitigen Anschlag (40) in die zweite Verschiebestellung verformt wird.

9. Vorrichtung nach Anspruch 8, **dadurch gekennzeichnet, dass** der Schieber (22) insgesamt elastisch ausgebildet ist.

10. Vorrichtung nach Anspruch 8 oder 9, **dadurch gekennzeichnet, dass** der Schieber (22) nach dem Entfernen des Spritzenkonus (36) aufgrund der Elastizität des Schiebers (22) in seine erste Verschiebestellung zurückkehrt.

11. Vorrichtung nach einem der Ansprüche 8 bis 10, **dadurch gekennzeichnet, dass** das Gehäuse (2) auf der dem Anschluss (5) zugewandten Seite eine Öffnung (21) zum Einführen des Schiebers (22) aufweist, und die der Öffnung (21) abgewandte Seite des Gehäuses (2) den Anschlag (40) für den Schieber (22) bildet.

12. Vorrichtung nach einem der Ansprüche 8 bis 11, **dadurch gekennzeichnet, dass** derjenige Bereich des Schiebers (22) elastisch ausgebildet ist, der dem Verbindungskanal (26) zugeordnet ist.

13. Vorrichtung nach einem der Ansprüche 1 bis 12, **dadurch gekennzeichnet, dass** der Schieber (22) abgedichtet im Gehäuse (2) geführt ist.

14. Vorrichtung nach Anspruch 13, **dadurch gekennzeichnet, dass** der Schieber (22) am Umfang einen Wulst (25) aufweist, der den Schieber (22) auf der dem Anschluss (5) abgewandten Seite des Gehäuses (2) zum Gehäuse abdichtet

15. Vorrichtung nach einem der Ansprüche 1 bis 14, **dadurch gekennzeichnet, dass** der Schieber (22) am Umfang einen Abschnitt (27) aufweist, der in der zweiten Verschiebestellung des Schiebers (22) den Strömungskanal (16) des ersten Einstechelements (13) verschließt.

16. Vorrichtung nach einem der Ansprüche 1 bis 15, **dadurch gekennzeichnet, dass** das Gehäuse (2) im Bereich der Einstechelemente (13, 14) zylindrisch angeordnete Aufnahmen (9) zum rastierenden Einstecken von Behältnissen (11, 12), insbesondere des Halses von Fläschchen, aufweist.

17. Vorrichtung nach einem der Ansprüche 1 bis 16, **dadurch gekennzeichnet, dass** sie im Strömungsweg von einem Einstechelement (14) zur seitlichen Öffnung (21) ein Filterelement (20) aufweist.

## Claims

1. Transfer device (1), in particular for medical fluids, with a housing (2), with a first needle-shaped or mandril-shaped piercing element (13) mounted in the housing (2), with a second needle-shaped or mandril-shaped piercing element (14) mounted in the housing (2), the two piercing elements (13, 14) pointing away from one another and having flow channels (16, 17), and moreover with a slide (22) which is arranged between the two piercing elements (13, 14), is mounted in the housing (2) and can be displaced with respect to the piercing elements (13, 14) such that, in a first position of displacement of the slide (22), a flow connection is established between the two piercing elements (13, 14), and, in a second position of displacement of the slide (22), a flow connection is established between one of the piercing elements (14) and a lateral opening (21) of the housing (2), wherein the housing (2) has, in the area of the lateral opening (21), a connector (5) for insertion of a syringe cone (36) of a syringe (37), and, when the syringe cone (36) is inserted into the connector (5), the front end (38) of the syringe cone (36) moves the slide (22) from the first position of displacement to the second position of displacement **characterized in that** the slide (22) has, in the area of its circumference, a connection channel (26) which extends at least over part of a circle and which, in the first position of displacement of the slide (22), connects the flow channels (16, 17) of the two piercing elements (13, 14) to one another and **in that** the slide (22) has, on the circumference, a removal channel (29) which extends in the longitudinal direction of the slide (22) and which, in the second position of displacement of the slide (22), is in flow connection with the flow channel (17) of one of the piercing elements (13, 14), the removal channel (29) being formed by an outer slit of the slide (22) and in particular having a V-shaped cross section.

2. Device according to Claim 1, **characterized in that** the connector (5) is designed as a female Luer connector.

3. Device according to Claim 1 or 2, **characterized in that** the connector (5) can be closed off by means of a closure element (7), and in particular can be closed off in a leaktight manner.

4. Device according to Claim 3, **characterized in that** the closure element (7) is designed as a closure cap which can be screwed onto the connector (5).

5. Device according to one of Claims 1 to 4, **characterized in that** the slide (22) is mounted in the housing (2) in a manner secure against twisting.

6. Device according to one of Claims 1 to 5, **characterized in that** the slide (22) is clamped in the housing (2) or, at least in its first position of displacement, is locked in the housing (2).

7. Device according to one of Claims 1 to 6, **characterized in that** the housing (2) has, on the side directed away from the connector (5), an opening (23) for insertion of the slide (22).

8. Device according to one of Claims 1 to 7, **characterized in that** the slide (22) is elastic, at least in a partial area (41), in particular made of a thermoplastic elastomer or of rubber, this partial area (41) being deformed when the slide (22) is moved from the first position of displacement into the second position of displacement against a stop (40) on the housing side.

9. Device according to Claim 8, **characterized in that** the whole slide (22) is elastic.

10. Device according to Claim 8 or 9, **characterized in that** the slide (22), because of its elasticity, returns to its first position of displacement after removal of the syringe cone (36).

11. Device according to one of Claims 8 to 10, **characterized in that** the housing (2), on the side directed toward the connector (5), has an opening (21) for insertion of the slide (22), and the side of the housing (2) directed away from the opening (21) forms the stop (40) for the slide (22).

12. Device according to one of Claims 8 to 11, **characterized in that** that area of the slide (22) allocated to the connection channel (26) is made elastic.

13. Device according to one of Claims 1 to 12, **characterized in that** the slide (22) is guided in a sealed manner in the housing (2).

14. Device according to Claim 13, **characterized in that** the slide (22) has, on the circumference, a bead (25) which seals the slide (22) off from the housing on that side of the housing (2) directed away from the connector (5).

15. Device according to one of Claims 1 to 14, **characterized in that** the slide (22) has, on the circumference, a portion (27) which, in the second position of displacement of the slide (22), closes the flow channel (16) of the first piercing element (13).

16. Device according to one of Claims 1 to 15, **characterized in that** the housing (2) has, in the area of the piercing elements (13, 14), cylindrically disposed seats (9) for lockable insertion of containers (11, 12), in particular of the neck of vials.

17. Device according to one of Claims 1 to 16, **characterized in that** it has a filter element (20) arranged in the flow path leading from one piercing element (14) to the lateral opening (21).

## Revendications

1. Dispositif de transfert (1), notamment pour fluides médicaux, comprenant un boîtier (2), un premier élément de piquage (13) en forme d'aiguille ou de poinçon monté dans le boîtier (2), un deuxième élément de piquage (14) en forme d'aiguille ou de poinçon monté dans le boîtier (2), les deux éléments de piquage (13, 14) étant orientés dans des directions opposées et comportant des canaux d'écoulement (16, 17), comprenant par ailleurs un tiroir (22) disposé entre les deux éléments de piquage (13, 14), logé dans le boîtier (2) et pouvant être déplacé en coulissement par rapport aux éléments de piquage (13, 14), une communication d'écoulement étant établie entre les deux éléments de piquage (13, 14), dans une première position de coulissement du tiroir (22), et une communication d'écoulement entre l'un des éléments de piquage (14) et une ouverture latérale (21) du boîtier (2) étant établie dans une deuxième position de coulissement du tiroir (22), le boîtier comprenant, au niveau de l'ouverture latérale (21), un raccord (5) permettant l'introduction d'un cône de seringue (36) d'une seringue (37), le tiroir (22) étant déplacé, par l'extrémité frontale (38) du cône de seringue (36), de la première position de coulissement jusque dans la deuxième position de coulissement lors de l'introduction du cône de seringue (36) dans le raccord (5),
**caractérisé en ce que** le tiroir (22) comporte, au niveau de sa périphérie, un canal de communication (26) s'étendant au moins sur un cercle partiel, canal qui, dans la première position de coulissement du tiroir (22), relie les canaux d'écoulement (16, 17) des deux éléments de piquage (13, 14) l'un à l'autre, et **en ce que** le tiroir (22) comporte, au niveau de sa périphérie, un canal de prélèvement (29) s'étendant dans le sens longitudinal du tiroir (22), qui, dans la deuxième position de coulissement du tiroir (22), se trouve en position de communication d'écoulement avec le canal d'écoulement (17) de l'un des éléments de piquage (13, 14), le canal de prélèvement (29) étant constitué par une fente extérieure du tiroir (22) et présentant une section transversale notamment en forme de V.

2. Dispositif selon la revendication 1, **caractérisé en ce que** le raccord (5) est réalisé sous forme d'un raccord de Luer femelle.

3. Dispositif selon la revendication 1 ou 2, **caractérisé en ce que** le raccord (5) peut être fermé au moyen d'un élément obturateur (7), notamment de façon étanche.

4. Dispositif selon la revendication 3, **caractérisé en ce que** l'élément obturateur (7) est réalisé sous forme d'un bouchon obturateur, qui peut être vissé sur le raccord (5).

5. Dispositif selon l'une des revendications 1 à 4, **caractérisé en ce que** le tiroir (22) est monté de façon immobile en rotation dans le boîtier (2).

6. Dispositif selon l'une des revendications 1 à 5, **caractérisé en ce que** le tiroir (22) est positionné coincé ou du moins enclenché dans sa première position de coulissement à l'intérieur du boîtier (2).

7. Dispositif selon l'une des revendications 1 à 6, **caractérisé en ce que**, du côté éloigné du raccord (5), le boîtier (2) présente une ouverture (23) permettant l'introduction du tiroir (22).

8. Dispositif selon l'une des revendications 1 à 7, **caractérisé en ce que** le tiroir (22) est réalisé pour être élastique au moins dans une zone partielle (41), et est notamment fait en un élastomère thermoplastique ou en caoutchouc, cette zone partielle (41) étant déformée lors du déplacement du tiroir (22) de la première position de coulissement vers la deuxième position de coulissement, contre une butée (40) aménagée dans le boîtier.

9. Dispositif selon la revendication 8, **caractérisé en ce que** le tiroir (22) est réalisé pour être élastique dans son ensemble.

10. Dispositif selon la revendication 8 ou 9, **caractérisé en ce qu'**après le retrait du cône de seringue (36), le tiroir (22) retourne dans sa première position de coulissement en raison de l'élasticité du tiroir (22).

11. Dispositif selon l'une des revendications 8 à 10, **caractérisé en ce que** le boîtier (2) comporte, du côté tourné vers le raccord (5), une ouverture (21) permettant l'introduction du tiroir (22), et que le côté du boîtier (2), qui est éloigné de l'ouverture (21), forme la butée (40) pour le tiroir (22).

12. Dispositif selon l'une des revendications 8 à 11, **caractérisé en ce que** la partie du tiroir (22), qui est associée au canal de communication (26), est réalisée pour être élastique.

13. Dispositif selon l'une des revendications 1 à 12, **caractérisé en ce que** le tiroir (22) est guidé de façon étanche à l'intérieur du boîtier (2).

14. Dispositif selon la revendication 13, **caractérisé en ce que** le tiroir (22) comporte, à sa périphérie, un bourrelet (25) qui assure l'étanchéité du tiroir (22) par rapport au boîtier (2), du côté du boîtier, qui est éloigné du raccord (5).

15. Dispositif selon l'une des revendications 1 à 14, **caractérisé en ce que** le tiroir (22) comporte, à sa périphérie, une section (27) qui, dans la deuxième position de coulissement du tiroir (22), obture le canal d'écoulement (16) du premier élément de piquage (13).

16. Dispositif selon l'une des revendications 1 à 15, **caractérisé en ce qu'**au niveau des éléments de piquage (13, 14), le boîtier (2) comporte des logements cylindriques (9) permettant l'insertion de récipients (11, 12), notamment l'insertion du goulot de flacons.

17. Dispositif selon l'une des revendications 1 à 16, **caractérisé en ce qu'**il comporte un élément filtrant (20) placé dans le chemin d'écoulement de l'un des éléments de piquage (14) vers l'ouverture latérale (21).
